Europäisches Patentamt

European Patent Office

Office européen des brevets

(11) Veröffentlichungsnummer: **0 082 114**
**B1**

# (12) EUROPÄISCHE PATENTSCHRIFT

(45) Veröffentlichungstag der Patentschrift: **29.07.87**

(21) Anmeldenummer: **82810526.2**

(22) Anmeldetag: **06.12.82**

(51) Int. Cl.⁴: **C 12 N 1/20, C 12 N 1/32, C 02 F 3/34 // C12R1/01**

(54) **Mikroorganismen des Genus Hyphomicrobium und Verfahren zum Abbau von Methylgruppen-haltigen Verbindungen in wässrigen Lösungen.**

(30) Priorität: **11.12.81 CH 7932/81**

(43) Veröffentlichungstag der Anmeldung:
**22.06.83 Patentblatt 83/25**

(45) Bekanntmachung des Hinweises auf die Patenterteilung:
**29.07.87 Patentblatt 87/31**

(84) Benannte Vertragsstaaten:
**AT BE CH DE FR GB IT LI LU NL SE**

(56) Entgegenhaltungen:

CHEMICAL ABSTRACTS, Band 82, Nr. 9, 3. März 1975, Seite 265, Spalte 1, Nr. 53966u, Columbus, Ohio, USA

CHEMICAL ABSTRACTS, Band 96, Nr. 15, 20. April 1982, Seite 341, Spalte 2, Nr. 118761j, Columbus, Ohio, USA

CHEMICAL ABSTRACTS, Band 96, Nr. 19, 10. Mei 1982, Seite 425, Spalte 1, Nr. 159034t, Columbus, Ohio, USA

CHEMICAL ABSTRACTS, Band 82, Nr. 9, 3. März 1975, Seite 263, Spalte 2, Nr. 53953u, Columbus, Ohio, USA

(73) Patentinhaber: **CIBA-GEIGY AG**
**Klybeckstrasse 141**
**CH-4002 Basel (CH)**

(72) Erfinder: **Ghisalba, Oreste, Dr.**
**Feldbergstrasse 91**
**CH-4057 Basel (CH)**
Erfinder: **Heinzer, Franz, Dr.**
**Chemin des minoux 17**
**CH-2900 Porrentruy (CH)**
Erfinder: **Küenzi, Martin, Dr.**
**Hüslimattstrasse 16**
**CH-4132 Muttenz (CH)**

Courier Press, Leamington Spa, England.

**Beschreibung**

Die vorliegende Erfindung betrifft neue fakultativ methylotrophe Mikroorganismen des Genus Hyphomicrobium, ein Verfahren zur mikrobiologischen Reinigung von wässrigen Lösungen durch Abbau von Dimethylphosphit, Trimethylphosphit oder Methylsulfatsalzen in Gegenwart dieser Mikroorganismen, die Zellmasse, welche von diesen Mikroorganismen hergestellt wird, sowie die Verwendung der verfahrensgemäss erhältlichen Zellmasse.

Unter dem Begriff "methylotrophe Mikroorganismen" versteht man solche Mikroorganismen, die auf Nährmedien wachsen, welche als Kohlenstoffquelle Verbindungen mit nur einem Kohlenstoffatom, z.B. Methanol, enthalten.

Unter dem Begriff "fakultativ methylotrophe Mikroorganismen" versteht man solche Mikroorganismen, die auf Nährmedien wachsen, welche als Kohlenstoffquelle Verbindungen mit einem Kohlenstoffatom, z.B. Methanol, und/oder Verbindungen mit mehreren Kohlenstoffatomen, z.B. Glucose, enthalten.

In der Literatur sind fakultativ methylotrophe Mikroorganismen beschrieben, welche in wässriger Lösung eine oder mehrere Verbindungen aus der folgenden Gruppe von organischen Verbindungen abbauen bzw. als Kohlenstoff- oder als Kohlenstoff- und Stickstoffquelle verwerten können: Methanol, Aethanol, Acetate, z.B. Natriumacetat, Formiate, z.B. Natriumformiat, Monosaccharide, z.B. Glucose, Disaccharide, z.B. Saccharose, Dimethylphosphit, Trimethylphosphit oder bestimmte Methylammoniumverbindungen, z.B. Methylammonium-, Aethylmethylammonium- oder Dimethylammoniumchlorid bzw. die freien Amine davon.

Solche Mikroorganismen sind in verschiedenen Stammsammlungen, z.B. in der American Type Culture Collection (ATCC), in der Deutschen Sammlung von Mikroorganismen (DMS) oder in der National Collection of Industrial Bacteria (NCIB), hinterlegt und in den von diesen Hinterlegungsstellen publizierten Katalogen aufgeführt.

Bei der grosstechnischen Produktion in der chemischen Industrie fallen wässrige Lösungen, z.B. Abwässer, an, welche diese Verbindungen und/oder Methylsulfate, z.B. Natriummethylsulfat, zum Teil in sehr hohen Konzentrationen als Verunreinigungen enthalten. Um Belastungen für die Umwelt durch diese Verbindungen bzw. Salze zu vermeiden, muss man solche Abwässer reinigen.

Dieses Problem wird durch die vorliegende Erfindung gelöst, welche neue fakultativ methylotrophe Mikroorganismen des Genus Hyphomicrobium und die davon abgeleiteten Mutanten betrifft, welche Dimethylphosphit, Trimetylphosphit und vor allem Methylsulfate, z.B. Natriummethylsulfat, abbauen können. Die vorliegende Erfindung betrifft ebenfalls ein Verfahren zur mikrobiologischen Reinigung von wässrigen Lösungen durch Abbau von Dimethylphosphit, Trimethylphosphit oder von Methylsulfatverbindungen oder Mischungen dieser Verbindungen in Gegenwart dieser Mikroorganismen, die Zellmasse, welche von diesen Mikroorganismen hergestellt wird, und die Verwendung der verfahrensgemäss erhältlichen Zellmasse.

Die vorliegende Erfindung betrifft in erster Linie Mikroorganismen des Genus Hyphomicrobium aus der Gruppe der folgenden Stämme: Hyphomicrobium MS 72 (NRRL—B—12573), MS 75 (NRRL—B—12572), MS 219 (NRRL—B—12571), MS 223 (NRRL—B—12570) und MS 246 (NRRL—B—12569) und die von diesen Stämmen abgeleiteten Mutanten ein Verfahren zur mikrobiologischen Reinigung von wässrigen Lösungen durch Abbau von Natriummethylsulfat.

Die neuen Stämme sind bei der Agricultural Research Culture Collection (NRRL) in Peoria, Illinois 61604, USA am 3. November 1981 hinterlegt worden und werden dort unter den weiter vorn genannten Hinterlegungsnummern aufbewahrt.

Isolierung der neuen Mikroorganismen

Die neuen Mikroorganismen stammen aus dem Klärschlamm einer Abwasserreinigungsanlage der Ciba-Geigy AG (ARA—CG) und aus einer Mülldeponie in Croglio, Kanton Tessin, Schweiz und sind in an sich bekannter Weise wie folgt isoliert worden:

Isolierungsmethode 1

Eine Abwasserprobe oder eine Klärschlammsuspension (1 g pro 10 ml sterilem Wasser) wird auf sterilem Natriummethylsulfat-haltigem Agar (auf der Basis von Nährlösung MV 7) plattiert. Die Nährlösung MV 7 enthält in einem Liter Wasser die folgenden Bestandteile: 2 g $NH_4NO_3$ (Stickstoffquelle), 1,4 g $Na_2HPO_4$, 0,6 g $KH_2PO_4$ (Puffer und Phosphorquelle), 0,2 g $MgSO_4 \cdot 7 H_2O$, 0,01 g $CaCl_2 \cdot 2 H_2O$, 0,001 g $FeSO_4 \cdot 7 H_2O$ und 1 ml Spurenelementlösung (bestehend aus je 20 mg/l $Na_2MoO_4 \cdot 2 H_2O$, $Na_2B_4O_7 \cdot 10 H_2O$, $MnSO_4 \cdot H_2O$, $ZnSO_4 \cdot H_2O$ und $CuSO_4 \cdot 5 H_2O$.

Man löst die Salze in destilliertem Wasser, bringt mit verdünnter Natronlauge auf pH 7 und füllt mit destilliertem Wasser auf 1 Liter auf. Zur Herstellung des festen Nährbodens MV 7-Agar werden der Nährlösung noch 20 g/l Agar (Difco) zugesetzt. Man sterilisiert im Autoklaven. Die Inkubation erfolgt bei 28—30°C. Einzelkolonien werden vorsichtig abgehoben und wieder auf dem gleichen Medium ausgestrichen. Man wiederholt diese Prozedur mehrmals, bis reine Isolate vorliegen.

2

Isolierungsmethode 2

Eine Abwasserprobe oder eine Klärschlammsuspension (1 g pro 10 ml sterilem Wasser) wird in einem Schüttelkolben gegeben, worin sich sterile Nährlösung MV 7 befindet. Man gibt eine sterilfiltrierte wässrige Natriummethylsulfat-Lösung hinzu und inkubiert bei 28°C als Standkultur 14 Tage oder als Schüttelkultur bei 250 Upm 7 Tage lang. 0,5 ml dieser ersten Anreicherungskultur werden in die frische Nährlösung MV 7 gegeben und wieder bei 28°C als Standkultur oder Schüttelkultur inkubiert. 0,5 ml der zweiten Anreicherungskultur werden wieder in die frische Nährlösung gegeben und 7 Tage bei 28°C inkubiert. Die zweite oder dritte Anreicherungskultur wird auf sterilem Natriummethylsulfat-haltigem MV 7-Agar [Nährlösung MV 7 mit Zusatz von 20 g/l Agar (Difco)] plattiert und bei 28°C inkubiert. Einzelkolonien werden vorsichtig abgehoben und wieder auf dem gleichen Medium ausgestrichen. Diese Prozedur wird mehrmals wiederholt, bis reine Isolate vorliegen.

In der folgenden Tabelle 1 sind Hinterlegungs-Nr., Herkunft und Isolierungsmethode für jeden einzelnen der weiter vorn genannten Stämme angegeben:

TABELLE 1

| Bezeichnung | | Hinterlegungs-Nr. (NRRL) | Herkunft | Methode |
|---|---|---|---|---|
| Genus | Kenn-Nr. | | | |
| Hyphomicrobium | MS 72 | B—12573 | ARA—CG | 1 |
| Hyphomicrobium | MS 75 | B—12572 | ARA—CG | 1 |
| Hyphomicrobium | MS 219 | B—12571 | ARA—CG | 2 |
| Hyphomicrobium | MS 223 | B—12570 | ARA—CG | 2 |
| Hyphomicrobium | MS 246 | B—12569 | Croglio | 2 |

Charakterisierung der neuen Mikroorganismen

1. Allgemeine Parameter und Mikroskopie

Alle in der Tabelle 1 aufgezählten Stämme sind gramnegativ, Oxidasepositiv und wachsen unter aeroben Bedingungen optimal bei 28° bis 30°. Als C-Quellen bzw. C- und N-Quellen können Sie in wässriger Lösung Methanol, Aethanol, Glucose, Dimethylphosphit, Trimethylphosphit, Formiat-, Acetat-, Methylammonium-, Dimethylammonium-, Aethylmethylammonium- oder Methylsufationen verwerten.

In der mikroskopischen Abbildung (Lichtmikroskop) sind sich alle Stämme sehr ähnlich und als motile Stäbchen von ca. 1—2μ Länge zu erkennen, welche einzeln paarweise oder agglomeriert auftreten. Viele Zellen tragen "Stiele" oder Hyphen. Das elektronenmikroskopische Bild befindet sich in guter Uebereinstimmung mit den lichtmikroskopischen Beobachtungen. Ein Teil der Zellen zeigt jeweils lange "Stiele" oder "Hyphen", wobei jede Zelle höchstens einen solchen Stiel in polarer Position besitzt. Einige gestielte Zellen traagen Geisseln. Bei einzelnen gestielten Zellen ist am Ende des Stiels eine Verdickung (Knospe) zu erkennen. Ein anderer Zelltyp besitzt zwar keinen Stiel, dafür aber eine subpolare Geissel.

Elektronenmikroskopische Abbildung einiger typischer Zellen von Stämmen gemäss Tabelle 1:

MS 75   18'500 x

MS 75   32'500 x          MS 75 32'500 x    Schwärmerzelle

2. Biochemische Charakterisierung und Klassifizierung der neuen Mikroorganismen

Zur Charakterisierung der in Tabelle 1 aufgezählten Stämme werden zwei käufliche Testsysteme verwendet. Beide Systeme eignen sich für allgemeine biochemische Charakterisierungen.

a) "Oxi-Ferm Tube"-Test (Roche)

Dieses Testsystem wird bei gramnegativen Stäbchen mit positiver Oxidasereaktion angewendet. Der Test wird parallel je einmal mit Impfmaterial des betreffenden Stamms ab Methanol-Agar und ab Nutrient-Agar durchgeführt. Experimentelle Ausführung nach Vorschrift des Herstellers. Die Testresultate sind in Tabelle 2 zusammengestellt.

TABELLE 2

MS—Stämme getestet mit "Oxi/Ferm Tube" (48 h, 28°C)

| Kenn-Nr. | anaerobe Dextrosespaltung | Arginin-dihydrolase | N$_2$-Produktion | H$_2$S-Bildung | Indol-Bildung | Xylosespaltung | aerobe Dextrosespaltung | Urease | Citratverwertung |
|---|---|---|---|---|---|---|---|---|---|
| | biochemischer Test | | | | | | | | |
| MS 72 | + | − | −* | − | − | + | + | + | ± |
| MS 75 | − | − | −* | − | − | − | − | − | − |
| MS 219 | − | − | + | − | − | ± | − | + | + |
| MS 223 | − | − | −* | − | − | − | − | − | − |
| MS 246 | − | − | + | − | − | ± | − | + | + |

+ beide Paralleltests positiv
− beide Paralleltest negativ
± (ein Test positiv, ein Test negativ
    (bei Xylosespaltung: ab Methanol-Agar positiv für MS 219 und
    246, bei Citratverwertung: ab Nutrient Agar positiv für MS 72)
* im API 20E-Test positiv

Anhand der Testresultate in Tabelle 2 lassen sich die einzelnen Stämme aufgrund ihrer übereinstimmenden Merkmale in drei Gruppen zusammenfassen:

5

TABELLE 3

| Kenn-Nr. | Uebereinstimmung | Gruppe |
|---|---|---|
| MS 72 | Einzelstamm | I |
| MS 75 und 223 | identisch | II |
| MS 219 und 246 | idenisch | III |

b) API 20 E-Test

Der Test wird parallel je einmal mit Impfmaterial des betreffenden Stamms ab Methanol-Agar und ab Nutrient-Agar durchgeführt. Experimentalle Ausführung nach Vorschrift des Herstellers. Die Testresultate sind in Tabelle 4 zusammengestellt.

TABELLE 4

MS—Stämme getestet mit API 20E (48 h, 28°C)

biochemischer Test

| Test | MS 72 | MS 75 | MS 219 | MS 223 | MS 246 |
|---|---|---|---|---|---|
| CAT: Katalase | – | + | + | + | + |
| N₂: Nitratreduktion | + | + | + | + | + |
| NO₂: | + | + | + | + | + |
| OX: Oxidase | +ˢ | +ˢ | + | + | + |
| ARA: L-Arabinoseverwertung | + | – | – | – | – |
| AMY: Amygdalinverwertung | + | – | – | – | – |
| MEL: Melibioserverwertung | + | – | – | – | – |
| SAC: Saccharoseverwertung | + | – | – | – | – |
| RHA: Rhamnoseverwertung | + | – | – | – | – |
| SOR: Sorbitverwertung | + | – | – | – | – |
| INO: Inositverwertung | + | – | – | – | – |
| MAN: Mannitverwertung | + | – | – | – | – |
| GLU: Glucoseverwertung | + | – | – | – | – |
| GEL: Gelatineverflüssigung | – | + | – | – | – |
| VP: Acetointest | + | ± | ± | ± | ± |
| IND: Tryptophanabbau → Indolbildung | – | – | – | – | – |
| TDA: Tryptophandesaminase | – | – | – | – | – |
| URE: Urease | –* | – | + | – | + |
| H₂S: Thiosulfatspaltung | – | – | – | – | – |
| CIT: Citratverwertung | + | – | + | – | + |
| ODC: Ornithindecarboxylase | + | – | – | – | – |
| LDC: Lysindecarboxylate | + | – | ± | – | – |
| ADH: Arginindihydrolase | – | – | – | – | ± |
| ONPG: Hydrolyse durch β-Galactosidase | + | + | + | + | + |
| Stamm Nr. | MS 72 | MS 75 | MS 219 | MS 223 | MS 246 |

+  beide Paralleltests positiv (s = schwach)
–  beide Paralleltests negativ
±  ein Test positiv, ein Test negativ (ADH, LDC ab Nutrient Agar positiv für MS 246 bzw. MS 219, VP ab Methanol-Agar positiv bei MS 75 bis MS 246)
*  im Oxi/Ferm Tube positiv

Oxidasetest (Cytochromoxidase) bei H₂S und ONPG Katalasetest bei MAN, INO und SOR

Nach den Testresultaten gemass Tabelle 4 lassen sich die einzeinen Stämme wiederum in die Gruppen I—III gemäss Tabelle 3 einordnen. Allerdings zeigen sich innerhalb der Gruppen kleine Unterschiede:
— MS 75 kann Gelatine verflüssigen, MS 223 nicht.
— MS 219 und MS 246 unterscheiden sich eventuell hinsichtlich ADH und LDC (zusätzlich neigt MS 246 zu flockigem Wachstum).

c) Vergleich der beiden Testsysteme a) und b)
Es lassen sich für die fünf Stämme gemäss Tabelle 1 folgende Quervergleiche anstellen:
$H_2S$-Bildung, Indolbildung und Arginindihydrolase in beiden Systemen immer negativ. Urease stimmt in beiden Systemen relativ gut und Citratverwertung stimmt in beiden Systemen vollständig überein. Die unterschiedlichen Befunde hinsichtlich $N_2$-Produktion (Nitratreduktion) werden mit der höheren Empfindlichkeit der Bestimmungen im API 20 E-Test erklärt.

Die Resultate des "Oxi/Term Tube" Tests und des "API 20 E" Tests würden eine Zuordnung der Stämme gemäss Tabelle 1 als Pseudomonas species, Pseudomonas-ähnlich, Alcaligenes faecalis oder Achromobacter species. erlauben. Neben biochemischen Kenndaten ist für diese Genera ausserdem die Art der Begeisselung der gramnegativen Stäbchen typisch. So sind Pseudomonas-Stämme monotrich oder multitrich polar begeisselt und in sehr seltenen Fällen unbegeisselt und Achromobacter mit 1 bis 4, gelegentlich bis 8, peritrichen Geisseln versehen. Der elektronenmikroskopische Befund für einige typische Zellen (siehe Abbildung vorn im Text) zeigt jedoch, dass die Stämme gemäss Tabelle 1 keinem der oben erwähnten Genera zugeordnet werden können. Die Stämme sind eindeutig gestiele Bakterien. Sowohl der lichtmikroskopische als auch der elektronenmikroskopische Befund passt zu der Obergruppe der knospenden oder gestielten Bakterien und innerhalb dieser Gruppe zum Genus Hyphomicrobium. Der elektronenmikroskopische Befund stimmt mit den in "Bergey's Manual of Determinative Bacteriology" (8. Auflage) im Abschnitt "Budding and/or appendaged Bacteria" für das Genus Hyphomicrobium beschriebenen Merkmalen überein.

Konservierung der Stämme
Für die Konservierung der Stämme gemäss Tabelle 1 eignen sich folgende Methoden:
a) Adsorption des Zellmaterials des betreffenden Stamms auf Glaskügelchen in Glycerinlösung und anschliessende Lagerung bei −20°C,
b) Aufbewahrung des Zellmaterials des betreffenden Stamms auf Schrägagar und
c) Lyo-Ampullen. Die betreffende Kultur wird von der Nährlösung abzentrifugiert und die Zellmasse in 1/4 bis 1/3-Volumen 15%iger Skim Milk resuspendiert und lyophilisiert.
Von der in der Tabelle 1 aufgezählten Stämmen können sich spontan Mutanten bilden oder es lassen sich künstlich Mutanten herstellen, die ebenso wie die natürlichen Stämme Dimethylphosphit, Trimethylphosphit sowie vor allem Methylsulfate, z.B. Natriummethylsulfat, in wässriger Lösung abbauen können und Zellmasse produzieren. Solche Mutanten kann man chemisch, z.B. mit gewissen Guanidinderivaten, z.B. N-Methyl-N'-nitro-N-nitrosoguanidin oder mit Alkalinitrit, z.B. Natriumnitrit, oder physikalisch, z.B. durch Ultraviolett-, Röntgen- oder radioaktive Strahlung, erzeugen.

Die erfindungsgemässen Mikroorganismen finden Anwendung in einem Verfahren zur mikrobiologischen Reinigung von wässrigen Lösungen durch Abbau von Dimethylphosphit, Trimethylphosphit oder von Methylsulfatverbindungen sowie Mischungen dieser Verbindungen, welches dadurch gekennzeichnet ist, dass man in einer diese Verbindungen enthaltenden wässrigen Lösung einen Mikroorganismus des Genus Hyphomicrobium oder eine von diesem Mikroorganismus abgeleitete Mutante, welche ebenfalls diese Verbindungen oder Salze abbauen kann, in Gegenwart von essenziellen anorganischen Salzen und gegebenenfalls einer Stickstoffquelle bei ca. 20° bis ca. 40° und einem pH-Wert von ca. 4 bis 7,5 züchtet und, wenn erwünscht, die erhältliche Zellmasse isoliert.

Bei der Züchtung oder Fermentation bauen die in Tabelle 1 aufgezählten Stämme die in wässriger Lösung, z.B. in einem Abwasser, befindlichen Bestandteile Dimethylphosphit, Trimethylphosphit, und vor allem Natriummethylsulfat ab und verbrauchen Sauerstoff.

Diese Bestandteile können zum Teil in sehr hohen Konzentrationen der betreffenden Verbindung oder des betreffenden Salzes, von den in Tabelle 1 aufgezählten Mikroorganismen abgebaut werden. Beim Abbauverfahren produzieren die Mikroorganismen Zellmasse und als weiteres Fermentationsprodukt Kohlendioxid. Beim Abbau von Natriummethylsulfat entsteht als weiteres Fermentationsprodukt Natriumhydrogensulfat. Der pH-Wert ist durch Zugabe von Pufferlösung, z.B. Phosphatpufferlösung, oder von wässriger Base, z.B. wässriger Natrium- oder Kaliumhydroxidlösung, auf Werte zwischen ca. 4,0 und 7,5, bevorzugt ca. 5 bis 6, einzustellen.

Die Züchtung erfolgt in Gegenwart von essenziellen anorganischen Salzen. Solche Salze sind beispielweise Halogenide, z.B. Chloride, Carbonate, Sulfate oder Phosphate von Alkali-, Erdalkali- oder Uebergangsmetallen, sowie Borate oder Molybdate vom Alkalimetallen.

Bevorzugte essentielle anorganische Salze sind beispielsweise Dinatrium- oder Dikaliumhydrogenphosphat, Natrium- oder Kaliumdihydrogenphosphat, Magnesium- oder Eisensulfat und Kalium- und Calciumchlorid. In geringen Mengen können noch Zink-, Mangan- und Kupfersulfat, Natriummolybdat und Borax zugestezt werden.

Als Stickstoffquelle setzt man beispeilsweise Aminosäuren, Peptide oder Proteine bzw. deren Abbauproduke wie Peton oder Trypton, Fleischextrakte, Mehle, z.B. von Mais, Weizen oder Bohnen, z.B. von der Sojabohne, Destillationsrückstände von der Alkoholherstellung, Hefeextrakte, und bevorzugt Ammoniumsalze, z.B. Ammoniumchlorid, oder Nitrate, z.B. Kalium- oder Ammoniumnitrat, hinzu.

Die Züchtung erfolgt unter aeroben Bedingungen, z.B. unter Sauerstoff- oder Luftzufuhr, und zwar unter Schütteln oder Rühren in Schüttelkolben oder Fermentern. Die Züchtung lässt sich in einem Temperaturbereich von ca. 20° bis ca. 40°C, bevorzugt bei ca. 27° bis ca. 30°C, durchführen.

Die Züchtung kann ansatzweise, z.B. durch ein- oder mehrmalige Zugabe von Nährlösung bzw. der entsprechenden Kohlenstoff- und Stickstoffquellen, oder kontinuierlich durch dauernde Zugabe von Nährlösung bzw. der entsprechenden Kohlenstoff- und Stickstoffquellen, erfolgen.

Verzugsweise züchtet man in mehreren Stufen, indem man sunächst eine oder mehrere Vorkulturen, z.B. in einem flüssigen Nährmedium, herstellt, welche man anschliessend in die eigentliche Hauptkultur überimpft. Eine Vorkultur lässt sich beispielsweise herstellen, indem man eine Probe mit Zellmaterial des betreffenden Mikroorganismus, der beispielsweise über Schrägagar aufbewahrt wird, in eine sterile Nährlösung, z.B. MV 7 mit einer geeigneten Kohlenstoffquelle, z.B. Natriummethylsulfat, überträgt und mehrere Tage bei 28° inkubiert. Mit dieser ersten Vorkultur impft man frische Nährlösung, z.B. MV 7, mit derselben Kohlenstoffquelle an und inkubiert nochmals mehrere Tage bei 28°.

Man kann den Fermentationsverlauf durch Probenentnahme analytisch während der Fermentation verfolgen, z.B. durch Messung des pH-Werts der Kultur oder der optischen Dichte, welche ein Mass für das Wachstum des jeweiligen Stamms ist, sowie gravimetrisch anhand des Trockengewichts der gebildeten Zellmasse.

Die gebildete Zellmasse lässt sich abschliessend z.B. nach einem der zahlreichen in der Europäischen Patentschrift 0 010 243 beschriebenen Verfahren aufarbeiten und in Düngemittel überführen.

Als Zellmasse sind im vorliegenden Fall alle sich in lebendem Zustand, z.B. im Teilungszustand oder Ruhezustand, im partiellen oder vollständigen Zelltod, oder bereits in der enzymatischen Zersetzung oder in der Zersetzung durch Fremdkulturen befindlichen Zellsysteme zu verstehen, welche von den Mikroorganismen der vorliegenden Anmeldung aufgebaut werden.

Diese Zellmasse ist ein wertvoller Rohstoff und kann als Einzellerprotein beispielsweise als Viehfutterzusatz verwendet werden. Die Zellmasse lässt sich auch beispielsweise als Suspension oder aufgearbeitet, z.B. nach Entwässerung oder Pasteurisierung, als Düngemittel verwenden. Mann kann die Zellmasse auch als Ausgangsmaterial zur Herstellung von Biogas mit hohem Heizwert (Zusammensetzung ca. 70% Methan, 29% Kohlendioxid und 1% Wasserstoff, Heizwert ca. 5500—6500 kcal/m$^2$), beispielsweise durch anaerobe Vergärung in Faultürmen verwenden. Der Rückstand (Faulschlamm) aus dem Herstellungsverfahren von Biogas ist ebenfalls ein hochwertiger Dünger, der im Vergleich zur ursprünglichen Zellmasse mit Stickstoff stark angereichert ist.

Die folgenden Beispiele illustrieren die vorliegende Erfindung. Die Temperaturangaben erfolgen in Grad Celsuis.

### Beispeil 1 (Herstellung der Vorkultur)

1 Probe mit über Schrägagar aufbewahrtem Zellmaterial des Mikroorganismus von Stamm MS 72 (siehe Tabelle 1) wird in einen Schüttelkolben enthaltend 20 ml Nährlösung MV 7 mit der vorn angegebenen Zusammensetzung und 5 g/l Natriummethylsulfat gegeben. Man setzt ca. 1mMol einer Phosphatpufferlösung von pH-7 hinzu und inkubiert 72 Stunden bei 28° und 250 Upm. 5—7 ml dieser ersten Vorkultur werden in einem zweiten Schüttelkolben enthaltend 100 ml Nährlösung MV 7, 10 g/l Natriummethylsulfat und 5 mMol Phosphatpuffer (pH: 7) 72 Stunden bei 28° und 250 Upm inkubiert.

### Beispiel 2

Analog Beispiel 1 lassen sich Vorkulturen der Stämme MS 75, 219, 223 und 246 (siehe Tabelle 1) herstellen.

### Beispiel 3

In einem Laborfermenter werden 10 l gegebenenfalls hitzesterilisierte (Sterilisation 20 Min. bei 120°) Nährlösung MV 7 mit einer gegebenenfalls sterilfiltrierten Natriummethylsulfatlösung so vereinigt, dass ein ungefähres Arbeitsvolumen von 10 l mit einer Konzentration von ca. 5 g/l Natriummethylsulfat resultiert.

Man gibt eine Probe mit ca. 500 ml der zweiten Vorkultur des Stamms MS 72 hinzu und hält folgende Bedingungen eine pH-Wert von 5,5, welcher durch Zugabe jeweils von 4-normaler Natronlauge und 1-normaler Salzsäure konstant gehalten wird, Temperatur: 28°, Luftzufuhr: 0.26 l/1 min. und Rührgeschwindigkeit von 400 bis 700 Upm.

Der Stamm wächst auf reinem Natriummethylsulfat als einziger Kohlenstoffquelle. Nach 75 Stunden ist das eingesetzte Natriummethylsulfat praktisch vollständig abgebaut. Die gebildete Zellmasse liegt als Gemisch von Einzelzellen oder Aggregaten verschiedener Grösse vor, welche man durch Absetzen oder Zentrifugieren abrennen kann.

9

Beispiel 4

Analog Beispiel 3 lässt sich Natriummethylsulfat in wässriger Lösung abbauen, indem man in einem Laborfermenter Vorkulturen der Stämme MS 75, 219, 223 und 246 züchtet.

Beispiel 5

In einem Laborfermenter werden 10 l hitzesterilisierte (Sterilisation 20 Min. bei 120°) Nährlösung MV 7 mit einer sterilfiltrierten Natriummethylsulfat-haltigen Abwasserlösung (Zusammensetzung: 31% Natriummethylsulfat, 10% Methanol, 1% Sulfat, 56% Wasser und weniger als 2% Aromaten) vereinigt, so dass ein Gesamtvolumen von ca. 10 l mit einer Konzentration von ca. 10 g/l Natriummethylsulfat resultiert. Man gibt eine Probe mit ca. 500 ml der zweiten Vorkultur des Stamms MS 72 hinzu und hält die im Beispiel 3 genannten Bedingungen ein. Innerhalb der nächsten 4—5 Tage erfolgt ein vollständiger Abbau des Substrats.

Beispiel 6

Analog Beispiel 5 lässt sich Natriummethylsulfat in sterilfiltrierten Abwasserlösungen abbauen, indem man in einem Laborfermenter Vorkulturen der Stämme MS 75, 219, 223 und 246 züchtet.

Beispiel 7

Analog Beispiel 5 und 6 lässt sich unter Verzicht auf Sterilfiltration Natriummethylsulfat in Abwasserlösungen abbauen, indem man in einem Laborfermenter Vorkulturen der Stämme MS 72, 75, 219, 223 und 246 züchtet.

**Patentansprüche**

1. Mikroorganismen des Genus Hyphomicrobium und davon abgeleitete Mutanten, dadurch gekennzeichnet, dass diese Mikroorganismen und deren Mutanten in wässriger Lösung Dimethylphosphit, Trimethylphosphit oder Methylsulfatsalze oder Mischungen davon abbauen und dabei Zellmasse produzieren können.

2. Mikroorganismen gemäss Anspruch 1 des Genus Hyphomicrobium und davon abgeleitete Mutanten, dadurch gekennzeichnet, dass diese Mikroorganismen und deren Mutanten in wässriger Lösung Natriummethylsulfat abbauen und dabei Zellmasse produzieren können.

3. Mikroorganismen gemäss Anspruch 1 oder 2 des Genus Hyphomicrobium aus der Gruppe der folgenden Stämme:
Hyphomicrobium MS 72 (NRRL—B—12573),
Hyphomicrobium MS 75 (NRRL—B—12572),
Hyphomicrobium MS 219 (NRRL—B—12571),
Hyphomicrobium MS 223 (NRRL—B—12570) und
Hyphomicrobium MS 246 (NRRL—B—12569)
und von diesen Stämmen abgeleitete Mutanten, welche in wässriger Lösung Dimethylphosphit, Trimethylphosphit oder Methylsulfatsalze oder Mischungen davon abbauen und dabei Zellmasse produzieren können.

4. Verfahren zur mikrobiologischen Reinigung von wässrigen Lösungen enthaltend Dimethylphosphit, Trimethylphosphit oder Methylsulfatsalze oder Mischungen davon, gekennzeichnet, dass man in wässriger Lösung Dimethylphosphit, Trimethylphosphit oder Methylsulfatsalze abbauende Mikroorganismen des Genus Hyphomicrobium oder von diesen Mikroorganismen abgeleitete Mutanten, welche ebenfalls diese Verbindungen oder Salze abbauen können, in Gegenwart von essenziellen anorganischen Salzen und einer Stickstoffquelle bei ca. 20° bis ca. 40° und einem pH-Wert von ca. 4,0 bis ca. 7,5 züchtet und, wenn erwünscht, die erhältliche Zellmasse isoliert.

5. Verfahren gemäss Anspruch 4 zur mikrobiologischen Reinigung von wässrigen Lösungen enthaltend Natriummethylsulfat, dadurch gekennzeichnet, dass man einen Mikroorganismus aus der Gruppe der folgenden Stämme:
Hyphomicrobium MS 72 (NRRL—B—12573, MX 75 (NRRL—B—12572), MS 219 (NRRL—B—12571), MS 223 (NRRL—B—12570) und MS 246 (NRRL—B—12569) züchtet.

6. Verfahren nach Anspruch 4 oder 5, dadurch gekennzeichnet, dass man die Züchtung bei 28°C durchführt.

7. Verfahren nach einem der Ansprüche 4—6, dadurch gekennzeichnet, dass man die Züchtung kontinuierlich durchführt.

**Revendications**

1. Microorganismes du genre Hyphomicrobium et mutants en dérivant, caractérisés en ce que ces microorganismes et leurs mutants peuvent décomposer en solution aqueuse le diméthylphosphite, le triméthylphosphite ou les sels de sulfate de méthyl ou des mélanges de ces composés et former en même temps une biomasse.

2. Microorganismes selon la revendication 1 du genre Hyphomicrobium et mutants en dérivant, caractérisés en ce que ces microorganismes et leurs mutants peuvent décomposer le méthylsulfate de sodium en solution aqueuse et former en même temps une biomasse.

3. Microorganismes selon la revendication 1 ou 2 du genre Hyphomicrobium du groupe des souches suivantes:

Hyphomicrobium MS 72 (NRRL—B—12573),
Hyphomicrobium MS 75 (NRRL—B—12572),
Hyphomicrobium MS 219 (NRRL—B—12571),
Hyphomicrobium MS 223 (NRRL—B—12570) et
Hyphomicrobium MS 246 (NRRL—B—12569)

et mutants dérivés de ces souches, qui peuvent décomposer en solution aqueuse le diméthylphosphite, le triméthylphosphite ou les sels de sulfate de méthyle ou des mélanges de ces composés et former en même temps une biomasse.

4. Procédé d'épuration microbiologique de solutions aqueuses contentant du diméthylphosphite, du triméthylphosphite ou des sels de sulfate de méthyle ou des mélanges de ces composés, caractérisé en ce qu'on cultive en solution aqueuse des microorganismes du genre Hyphomicrobium dégradant le diméthylphosphite, le triméthylphosphite ou les sels de sulfate de méthyl ou des mutants dérivés de ces microorganismes qui peuvent également dégrader ces composés ou sels, en présence de sels minéraux essentiels et d'une source d'azote à une température comprise entre environ 20° et 40° et à un pH compris entre environ 4,0 et environ 7,5 et en ce qu'on isole, si on le désire, la biomasse obtenue.

5. Procédé selon la revendication 4 pour l'épuration microbiologique de solutions aqueuses contentant du méthylsulfate de sodium, caractérisé en ce qu'on cultive un microorganisme du groupe des souches suivantes:

Hyphomicrobium MS 72 (NRRL—B—12573), MS 75 (NRRL—B—12572), MS 219 (NRRL—B—12571), MS 223 (NRRL—B—12570), et MS 246 (NRRL—B—12569).

6. Procédé selon la revendication 4 ou 5, caractérisé en ce que la culture est réalisée à 28°C.

7. Procédé selon l'une des revendications 4 à 6, caractérisé en ce que la culture est réalisée en continu.


**Claims**

1. Microorganisms of the genus Hyphomicrobium or mutants derived therefrom, which microorganisms and their mutants, in aqueous solution, are able to degrade dimethyl phosphite, trimethyl phosphite or methyl sulfate salts or mixtures thereof, thereby producing biomass.

2. Microorganisms of the genus Hyphomicrobium according to claim 1, or mutants derived therefrom, which microorganisms and their mutants, in aqueous solution, are able to degrade sodium methyl sulfate, thereby producing biomass.

3. Microorganisms of the genus Hyphomicrobium according to either of claims 1 or 2, selected from the group of the following strains:

Hyphomicrobium MS 72 (NRRL—B—12573),
Hyphomicrobium MS 75 (NRRL—B—12572),
Hyphomicrobium MS 219 (NRRL—B—12571),
Hyphomicrobium MS 223 (NRRL—B—12570) and
Hyphomicrobium MS 246 (NRRL—B—12569)

or mutants derived from said strains, which microorganisms and their mutants, in aqueous solution, are able to degrade dimethyl phosphite, trimethyl phosphite or methyl sulfate salts or mixtures thereof, thereby producing biomass.

4. A process for the microbiological purification of aqueous solutions containing dimethyl phosphite, trimethyl phosphite or methyl sulfate salts or mixtures thereof, which process comprises culturing in aqueous solution microorganisms of the genus Hyphomicrobium which are capable of degrading dimethyl phosphite, trimethyl phosphite or methyl sulfate salts, or mutants which are derived from said microorganisms and are likewise capable of degrading these compounds or salts, in the presence of nutrient inorganic salts and a nitrogen source, in the temperature range from about 20° to about 40°C and in a pH range from about 4.0 to about 7.5, and, if desired, isolating the biomass obtained.

5. A process according to claim 4 for the microbiological purification of aqueous solutions containing sodium methyl sulfate, which process comprises culturing a microorganism selected from the group of the following strains:

Hyphomicrobium MS 72 (NRRL—B—12573), MS 75 (NRRL—B—12572), MS 219 (NRRL—B—12571), MS 223 (NRRL—B—12570) and MS 246 (NRRL—B—12569).

6. A process according to either of claims 4 or 5, wherein culturing is carried out at 28°C.

7. A process according to any one of claims 4 to 6, wherein culturing is carried out continuously.